# FASCICULE DE BREVET EUROPEEN

(11) **EP 3 380 660 B1**
(45) Date de publication et mention de la délivrance du brevet: **16.02.2022**
(21) Numéro de dépôt: 16815888.9
(22) Date de dépôt: 25.11.2016
(51) Int. Cl.: D04B 21/18

(54) **BANDE DE CONTENTION OPTIMISEE ET KIT UTILISANT LADITE BANDE**
OPTIMIERTES KOMPRESSIONSBAND UND ANWENDUNGSSATZ MIT DIESEM BAND
OPTIMISED COMPRESSION TAPE AND A KIT USING SAME

(30) Priorité: 26.11.2015 FR 1561402
(43) Date de publication de la demande: 03.10.2018
(73) Titulaire: Urgo Recherche Innovation et Développement, 21300 Chenove (FR)
(72) Inventeur: COHADE, Céline, 42650 Saint-Jean-Bonnefonds (FR); GRANGE, David, 42210 Bellegarde en Forez (FR); LECOMTE, Serge, 21000 Dijon (FR); ROBLOT, Magali, 21160 PERRIGNY-LES-DIJON (FR)
(74) Mandataire: Cabinet Beau de Loménie
(86) Numéro de dépôt international: PCT/FR2016/053105
(87) Numéro de publication internationale: WO 2017/089731

(56) Documents cités:
- WO-A1-95/16416
- US-A- 5 385 036

## Description

### DOMAINE TECHNIQUE GENERAL

La présente invention est relative à une bande de contention optimisée, dont l'allongement longitudinal est compris entre 30% et 160%, qui est un tricot 3D, obtenu selon la technologie « maille jetée », sans latex ni adhésif, qui ne se détend pas ce qui permet de conserver son efficacité thérapeutique et d'éviter son glissement au cours du temps.

### ETAT DE L'ART

L'utilisation de divers systèmes de contention est connue pour traiter les pathologies d'origine veineuse, comme par exemple l'insuffisance veineuse, le traitement des varices et des ulcères de jambes ou encore pour prévenir la thrombose veineuse ou le traitement des lymphœdèmes. Ces systèmes sont constitués d'une ou de plusieurs bandes qui appliquent une pression sur le membre à traiter.

Pour être efficace ce système doit permettre d'appliquer simultanément :
- d'une part, une pression relativement faible appelée « pression de repos », quand le muscle est relâché pour être conformable et en particulier supportable durant la nuit ; et
- d'autre part, une pression relativement élevée appelée « pression de travail », quand le muscle est tendu ou lors des mouvements, en particulier pendant la marche.

Ce différentiel de pression entre pression de travail et pression de repos doit être suffisant pour favoriser le reflux veineux. On considère généralement qu'un différentiel de pression à 24 heures compris entre 15 et 25 mm de mercure est nécessaire pour rétablir un flux veineux correct.

Toutefois selon la pathologie, qu'il s'agisse d'un traitement sur des jambes sans ulcères graves, d'un traitement difficile sur des jambes abimées par un œdème, ou d'un traitement d'un ulcère mixte artériel et veineux, cette plage de valeurs peut s'étendre de 10 à 35 mm de mercure voire même de 10 à 40 mm de mercure.

Les bandes de contention utilisées sont classées par les spécialistes de la contention en deux grandes catégories selon la mesure de leur allongement ; les bandes dites à allongement courts et celles dites bandes à allongement long.

Cette classification est basée sur la mesure de l'allongement longitudinal de la bande telle que définie dans la méthode A §9.1 de la norme EN 14704-1 quand la bande est soumise à une force de traction maximale de 6 N/cm.

Les conditions de réalisation de la mesure sont les suivantes.

Une éprouvette du matériau à tester de 50 mm de largueur et de 250 à 300 mm de longueur est découpée et positionnée sans précontrainte dans les mors d'un dynamomètre électronique (par exemple un dynamomètre de marque MTS) de sorte à avoir une largeur de 50 mm et une longueur utile de référence de 200mm. Le dynamomètre étire l'éprouvette à une vitesse de 100 mm/min jusqu'à une force maximale de 6 N/cm puis la traverse revient à sa position initiale à la même vitesse de retour de 100 mm/min. Ce cycle est effectué 5 fois et l'allongement obtenu au cinquième cycle, exprimé en pourcentage, est directement calculé par l'appareillage. L'opération est répétée sur 5 éprouvettes, puis on calcule la valeur moyenne qui définit l'allongement longitudinal de la bande.

L'allongement transversal de la bande peut être évalué selon le même protocole.

### Les bandes à allongement court

Sur la base de ce test selon la norme EN 14704-1 pris comme référence, on considère qu'une bande de contention est une bande à «allongement court» si son allongement longitudinal est inférieur ou égal à 100%.

Ces bandes exercent une pression au repos basse et une pression de travail élevée. Elles ont donc un grand différentiel de pression en particulier lors des mouvements, par exemple pendant la marche.

### Les bandes à allongement long

Sur la base du test précédent selon la norme EN 14704-1 pris comme référence, on considère qu'une bande est une bande à «allongement long» si son allongement longitudinal est supérieur à 100%.

Ces bandes sont plus faciles à poser car elles présentent une plus grande extensibilité.

Les bandes à allongement long conduisent à de faibles variations de pression entre repos et travail, et à une faible variation de pression au cours des mouvements, par exemple lors de la marche. Elles s'avèrent moins efficaces que les bandes à allongement court. En revanche du fait de ce faible différentiel de pression elles sont soumises lors des mouvements à des contraintes de force plus faibles que les bandes à allongements court, et elles présentent donc un risque de se détendre et donc de glisser le long de la jambe plus faible que les bandes à allongement court.

Il est reconnu aujourd'hui que les systèmes de contention les plus performants en termes de facilité et de rapidité de pose et en termes d'efficacité thérapeutique sont ceux qui comprennent au maximum 2 bandes et au moins une bande de contention dite à allongement court.

A titre d'exemple on peut citer les produits commercialisés sous les dénominations ACTICO, K2 et Coban 2 respectivement par les sociétés ACTIVA, Laboratoires URGO et 3M.

Le système ACTICO est constitué d'une bande à allongement court autoadhérente qui est enroulée sur une bande de ouate préalablement enroulée sur la jambe. La ouate est destinée à répartir les pressions à la surface du membre, et/ou à protéger par son épaisseur les saillies osseuses, et à absorber les éventuels exsudats si la bande est posée sur une plaie ouverte, par exemple dans le cas des ulcères de jambes.

Le système K2^{®} commercialisé par la société Laboratoires URGO est constitué d'une première bande (commercialisée sous la dénomination Ktech^{®}) qui est une bande à allongement court constituée d'une couche d'ouate venant au contact de la peau et aiguilletée à un tricot élastique et d'une seconde bande (commercialisée sous la dénomination KPress^{®}) élastique et autoadhérente qui est une bande à allongement long qui sert à maintenir la première bande en place et à appliquer la pression complémentaire par rapport à la première bande pour obtenir la pression recherchée.

Le système Coban 2 est constitué d'une première bande posée sans extension qui est formée de l'association d'une mousse venant au contact de la peau associée à une bande autoadhèrente et d'une seconde bande autoadhèrente qui est une bande à allongement court qui applique la pression recherchée et sert à maintenir le système.

Un inconvénient de ces différents systèmes est que pour garantir leur maintien et leur efficacité l'autoadhérence des bandes est obtenue à l'aide d'adhésif ou de latex ce qui complique leur mise au point et peut entraîner des risques d'allergie au contact de la peau, en particulier dans le cas du latex de caoutchouc naturel.

Le rôle de l'adhésif ou du latex est cependant incontournable car c'est lui qui permet de maintenir la bande ou le système après son enroulement autour d'un membre et de diminuer leur relâchement intrinsèque qui conduit à leur perte d'efficacité et leur glissement au cours du temps le long du membre.

En revanche l'incorporation de l'adhésif ou du latex complexifie la réalisation des produits car il modifie les propriétés de pression et de différentiel de pression de la bande sur laquelle il est appliqué.

Afin d'améliorer l'acceptabilité par les patients et le personnel soignant et d'obtenir un produit plus facile à fabriquer il apparait donc souhaitable de disposer d'un système de contention qui utilise des bandes sans adhésif ou latex.

Les tricots dits 3D sont des produits qui se présentent sous la forme de 2 surfaces textiles (des tricots) indépendantes reliées entre elles par des fils d'entretoises d'où leur nom de « 3D ». De tels produits sont par exemple utilisés dans le domaine des sièges automobiles pour leur capacité de compression. Mais pour obtenir cette capacité ces tricots sont épais, rigides et très élastiques. De même ils sont utilisés dans le domaine textile par exemple pour les bonnets de soutien- gorge. Ces tricots sont très doux au toucher mais à nouveau très élastique pour assurer le maintien. Dans les 2 cas ils ne sont pas adaptés pour remplir les propriétés d'une bande de contention.

D'autres tricots 3D adaptés pour la contention ont été proposés dans la demande de brevet WO 95 / 16416.

Le problème que propose de résoudre cette demande WO 95 /16416 est la suppression de la ouate. En effet le tassement de cette dernière au cours du temps induit un jeu entre la jambe et la bande lors des mouvements ce qui peut provoquer le glissement de l'ensemble. L'objectif est de compenser ce phénomène de tassement grâce à la structure 3D et à l'épaisseur du tricot qui permettent d'obtenir un bon effet amortisseur (padding) et de supprimer la ouate. Pour atteindre ce résultat les tricots 3D décrits présentent des grammages et des épaisseurs élevées. Ceci conduit à la réalisation de bandes de contention plus volumineuses donc moins facile à manipuler car elles se présentent sous forme de bobineaux plus épais. Elles sont aussi plus lourdes ce qui augmentent le risque quelles glissent plus facilement au cours du temps.

Ainsi c'est pour palier ce défaut et l'absence de latex ou d'adhésif que le document WO 2009 / 71894 propose d'incorporer un adhésif ou un latex aux tricots 3D proposés dans le document WO 95 / 16416, ce qui pose donc également les problématiques précitées vis-à-vis de l'adhésif ou du latex en termes de mise au point et de risques d'allergie.

La demande de brevet GB 2473321 propose la réalisation de tricots 3D toujours avec des grammages élevés pour s'approcher du rôle amortisseur de la ouate tout en appliquant des pressions et différentiels de pressions adaptés aux objectifs thérapeutiques. Toutefois tous les tricots décrits sont fabriqués selon la technologie « maille cueillie ». Du point de vue fabrication industrielle cette technologie n'est pas adaptée à la réalisation de bande de contention car la découpe d'une nappe de tricots 3D conduit au démaillage du produit. Le problème de glissement au cours du temps se pose également vis-à-vis de produits obtenus conformément à l'enseignement de ce document.

Pour y remédier, le document GB 2473321 propose de réaliser la « cohésivation » des tricots 3D par ajout de dérivés de silice seuls ou en association avec du latex ou des acrylates pour assurer le maintien au cours du temps, comme mentionné en page 14 de ce document, ce qui pose également les problématiques précitées en termes de mise au point et de risques d'allergie.

Le glissement d'une bande est causé par 3 facteurs primordiaux.

Le premier facteur est lié à la qualité de la pose.

Si une bande est posée à un allongement trop faible elle risque de glisser car la pression appliquée sur le membre sera insuffisante pour la tenir en place. Un dispositif d'étalonnage permet de résoudre ce problème et d'éviter qu'à l'inverse si la bande est trop étirée on applique une pression trop élevée qui pourrait conduire à former un garrot. De même il est nécessaire de bien fixer la dernière spire pour éviter que la bande se détende à son extrémité puis sur la totalité de son enroulement ce qui va entrainer une perte de son efficacité thérapeutique voire son glissement le long de la jambe. Divers dispositifs sont utilisés pour renforcer cette fixation.

Le deuxième facteur est lié à la capacité de la bande à résister au glissement sur la peau qui est dépendant de son état de surface venant en contact avec cette dernière. Cet aspect est difficile à compenser car on souhaite avoir une face en contact avec la peau qui présente un toucher le moins désagréable possible pour favoriser l'observance du port de la bande par le patient.

Le troisième facteur est lié au mode de fonctionnement de la bande. Il consiste à trouver un équilibre entre la force appliquée par la bande en extension lors de la variation du diamètre du mollet et sa capacité à éviter le glissement latéral spire sur spire qui est illustré par leur relâchement intrinsèque et qui traduit le fait qu'elles se détendent par rapport à la pose. On retrouve alors le même phénomène que lors d'une pose déficiente c'est-à-dire une perte d'efficacité thérapeutique voire en cas de transmission de ce glissement latéral de spire en spire au cours du temps un glissement vertical de la bande qui peut à nouveau conduire à sa chute. Ce phénomène est amplifié par le poids de la bande.

Ce troisième facteur, cause de glissement, est particulièrement important et représente aussi la principale raison de la perte d'efficacité des systèmes de contention au cours du temps.

Paradoxalement cette cause de glissement n'a pas été étudiée de façon approfondie jusqu' à présent. Pour résoudre ce problème et s'opposer à ce relâchement intrinsèque, on a «cohésivé » les bandes c'est-à-dire privilégié l'incorporation d'adhésif ou du latex sur les bandes de contention. Ainsi dans tous les systèmes de contention à base de bandes à allongement court pour lesquelles ce phénomène est le plus important on incorpore aujourd'hui au moins une bande « cohésivée », posant à nouveau les problématiques définies précédemment.

En l'absence de cohésivation ou de tout autre moyen pour éviter ce phénomène de glissement si les 2 premiers facteurs sont maitrisés le troisième facteur devient alors essentiel .L'efficacité thérapeutique et le relâchement intrinsèque de la bande qui augmente la possibilité de glissement sont étroitement liés à cet équilibre et son évolution au cours du temps.

En conclusion bien que l'utilisation d'un tricot 3D comme bande de contention ait été proposée depuis bientôt 20 ans aucune solution n'apparait totalement satisfaisante pour obtenir un tricot 3D qui présente le comportement d'un allongement court pour permettre d'obtenir le bon différentiel de pression et, qui permette en l'absence de latex ou d'adhésif, de le conserver et d'éviter le risque de glissement le long du membre au cours du temps.

Pour résoudre ce cahier des charges très complexe avec des propriétés contradictoires le déposant a étudié les forces de frottement qui s'appliquent au niveau d'une bande en contact avec elle-même sous l'effet d'une pression correspondant à la pression de traitement thérapeutique recherchée par exemple dans le cas d'un ulcère de jambe de l'ordre de 35 à 50 mm de mercure. En effet le glissement des spires de la bande est lié aux micro-déplacements de cette dernière sur elle- même imposés par son poids à cause de la pesanteur et aux efforts de friction répétitifs induits par les variations du diamètre du mollet lors des mouvements.

Pour effectuer la mesure, qui n'avait jamais été envisagée, de ces micro-déplacements de la bande sur elle-même qui sont très faibles le déposant a utilisé un rhéomètre qui est un appareil qui classiquement sert à mesurer les propriétés rhéologiques de matériaux mous. Cet appareillage outre qu'il permet de déterminer des forces très faibles permet aussi d'appliquer un couple de cisaillement c'est-à-dire une torsion afin d'être représentatif des contraintes de frictions qui s'appliquent sur une bande à la fois dans les sens longitudinal et dans le sens transversal de cette dernière. La technique ainsi mise au point a permis de déterminer l'effort de cisaillement minimum responsable du premier micro-déplacement que subit la bande enroulée sur elle- même qui va conduire au glissement latéral des spires et au relâchement de la bande. Cet effort de cisaillement est appelé contrainte seuil de cisaillement car elle mesure le premier micro-déplacement et est exprimé en Pascal.

Cette mesure a permis de mieux comprendre les phénomènes mis en jeu et de déterminer les caractéristiques essentielles que doit présenter un tricot 3D pour remplir les propriétés énumérées ci-dessus et en particulier la contrainte seuil de cisaillement qu'il doit posséder pour éviter le relâchement intrinsèque de la bande pour conserver son efficacité thérapeutique et ne pas glisser.

La présente invention est donc relative à une bande dont l'allongement longitudinal est compris entre 30% et 160%, qui est un tricot 3D, obtenu selon la technologie « maille jetée », sans latex ni adhésif, qui ne glissent pas au moins pendant 48 heures et mieux au moins pendant 3 jours ou plus. En effet dans le cadre du traitement des ulcères de jambes qui présentent des plaies très exsudatives ces durées minima de 48 et 72 heures correspondent aux durées habituelles de changement des pansements qui sont disposés sous les bandes de contention. Il est donc indispensable que la bande reste en place au moins 2 ou 3 jours sans glisser.

### PRESENTATION DE L'INVENTION

La présente invention est donc relative à une bande de contention qui se présente sous la forme d'un tricot obtenu par la technologie maille jetée, à base de fils synthétiques qui est constitué de 2 surfaces textiles dont la structure textile est identique ou différente reliées entre elles par des fils d'entretoises, chaque surface textile comportant des fils élastiques, ledit tricot présentant un allongement longitudinal mesuré selon la norme EN 14704 -1 compris entre 30 et 160% et une contrainte de seuil de cisaillement supérieure ou égale à 2800 Pa.

Des tests décrits ci - après ont démontré qu'une bande possédant comme caractéristique une telle contrainte de seul de cisaillement permet de s'assurer du non glissement spire sur spire de la bande de contention et ainsi d'éviter son relâchement intrinsèque et donc de conserver son efficacité thérapeutique et d'empêcher son glissement.

Selon la présente invention le tricot 3D pourra être à usage unique ou réutilisable et donc par conséquent lavable.

Après l'opération de tricotage, afin de stabiliser le tricot 3D, notamment afin d'obtenir un produit lavable, sa structure sera figée en utilisant les technologies couramment employées à cet effet telle la thermofixation par la chaleur ou un traitement de vaporisage. Ces opérations consistent à faire passer dans une étape supplémentaire, en ligne avec le tricotage ou séparée de cette dernière, à une vitesse donnée et une température fixée le tricot dans un four pour la thermofixation et à travers un flux de vapeur d'eau pour le vaporisage.

Afin de favoriser une pose précise par le personnel soignant, la bande de contention peut être munie d'un moyen d'étalonnage. Ce moyen d'étalonnage peut être visuel, comme par exemple un ensemble de pictogrammes, régulièrement espacés, imprimés sur la bande ou réalisés au moyen d'un système d'étalonnage. Des informations sur les allongements à la pose recommandés peuvent être fournies avec le moyen d'étalonnage. L'étalonnage peut aussi être réalisé par le personnel soignant sous la forme d'un pochoir. Ce type de pochoir ou les explications nécessaires pour le fabriquer peuvent être incorporés dans le conditionnement de la bande. Un kit comprenant plusieurs bandes de constitutions différentes, de largeurs différentes, de longueurs différentes et/ou dotées d'étalonnages différents pour appliquer des pressions spécifiques pourra être utilisé.

Le kit pourra en outre comprendre un ou plusieurs pansements destinés à être posés sur la plaie avant la pose de la bande.

Pour favoriser la facilité de manipulation lors de la pose on choisira un tricot qui présente un allongement longitudinal tel que défini dans la norme EN 14704-1 qui soit compris entre 40 et 160%, ou plus précisément entre 50 et 120 %, ou encore plus précisément entre 55 et 100%.

Le tricot présente par exemple une épaisseur comprise entre 1 et 2 mm, ou plus précisément entre 1 et 1,5mm.

Le tricot présente par exemple un grammage compris de 160 à 370 g/m², ou plus précisément de 180 à 300 g/m², ou encore plus précisément de 200 à 250 g/m².

De même de préférence le tricot présente par exemple un écart entre les 2 faces textiles compris entre 0,4 et 1,5mm, ou plus précisément entre 0,5 et 1,1mm.

Ces propriétés de faible grammage et d'épaisseur assurent une utilisation facilitée avec les chaussures de la bande de contention. La bande de contention pourra ainsi être aussi plus facilement utilisée avec une ouate si nécessaire.

Les deux surfaces textiles du tricot peuvent avoir des structures textiles identiques ou différentes. Ces structures textiles peuvent être pleines ou ajourées.

Les structures textiles ajourées appelées tricot à jours et désignées dans la présente demande sous le terme de filet sont bien connues par l'homme du métier. Un tricot à jours est un tricot qui présente des trous réguliers ou irréguliers dans sa structure textile. Ces trous sont obtenus lorsque, dans la structure textile, une ou plusieurs mailles d'une colonne ne sont pas reliées aux mailles de la colonne voisine lors du tricotage, typiquement en jouant sur le schéma de maille et / ou sur l'enfilage.

Selon un aspect de la présente invention le tricot présente deux surfaces textiles dont la structure textile est différente et en particulier une surface textile qui présente une structure textile ajourée appelée face filet et une surface textile qui présente une structure textile face pleine. La présence d'une face filet permet de favoriser la respirabilité de la bande. Une telle face filet est typiquement disposée au contact de la peau d'un utilisateur.

Selon un mode de réalisation particulier, ledit tricot présente une face qui a une structure textile de type drap, demi-simple à mailles ouvertes ou fermées, atlas sous un ou plusieurs rangs, ou chainette à mailles ouvertes, fermées, ou à alternance de mailles fermées et ouvertes. Cette face est opposée à la face adaptée pour être mise en contact avec la peau, qui présente une structure textile qui est un filet avec le même type ou un type différent de structure textile mais ajourée.

Afin de faciliter le passage du talon et éviter une striction de la bande lors de la pose on peut utiliser des tricots 3D qui présentent un allongement transversal supérieur à 120 % tel que mesuré selon la méthode A §9.1 de la norme EN 14704-1, ou par exemple compris entre 120% et 300%, ou encore entre 120% et 250%.

Les tricots selon l'invention sont par exemple réalisés à l'aide de fils couramment employés dans la réalisation des produits textiles et en particulier des tricots. Ces fils sont par exemple synthétiques. Ces fils se partagent en 2 grandes catégories les fils élastiques et les fils thermoplastiques.

Parmi les fils élastiques on peut par exemple citer les fils à base de fibres polyuréthanne comme les fils d'Elasthanne commercialisé sous la dénomination LYCRA, les fils à base d'élastodiène ou les fils à base de polymères triblocs (styrène - éthylène - butylène - styrène).

Parmi les fils thermoplastiques on peut citer les fils constitués à base de matériaux synthétiques qui ne sont pas des élastomères comme par exemple le polyester, le polyamide, le polypropylène, le polybutylène terephtalate (PBT).

Tous ces fils thermoplastiques peuvent être guipés ou non, texturés ou non.

Les deux surfaces textiles du tricot 3D sont par exemple réalisées à partir de fils élastiques et de fils thermoplastiques. Ces fils peuvent être monofilaments ou multifilaments. Ces surfaces textiles pourront être réalisées à partir de fils identiques ou différents. Les deux surfaces comprendront de préférence des fils élastiques similaires.

Les fils élastiques présents sur ces surfaces textiles présentent par exemple des titres de l'ordre de 40 à 80 dtex et les fils thermoplastiques des titres de 40 à 90 dtex.

Si l'on souhaite favoriser le transfert d'humidité du tricot vers l'extérieur on pourra utiliser des fils de nature non synthétique, comme par exemple le coton ou la viscose, sur une des deux faces en particulier celle en contact avec la peau.

On utilise par exemple comme fil élastique des fils d'élasthanne et comme fil thermoplastique des fils de polyamide ou de polyester.

Les fils d'entretoises sont typiquement des fils thermoplastiques monofilament, comme par exemple des fils en polyester ou en polyamide qui présentent par exemple un titre compris entre 20 et 80 dtex , ou encore entre 40 et 70 dtex, ou encore plus précisément un monofilament en polyester qui présente un titre entre 44 et 55 dtex.

Pour la réalisation du tricot 3D, on peut par exemple utiliser une seule barre pour tricoter le fil d'entretoise qui lie les 2 surfaces textiles.

L'invention concerne également un kit comprenant une ou plusieurs bandes de contention telles que définies précédemment, et un ou plusieurs pansements adaptés pour être disposés sur une plaie préalablement à l'une des bandes de contention.

### DESCRIPTION DETAILLEE

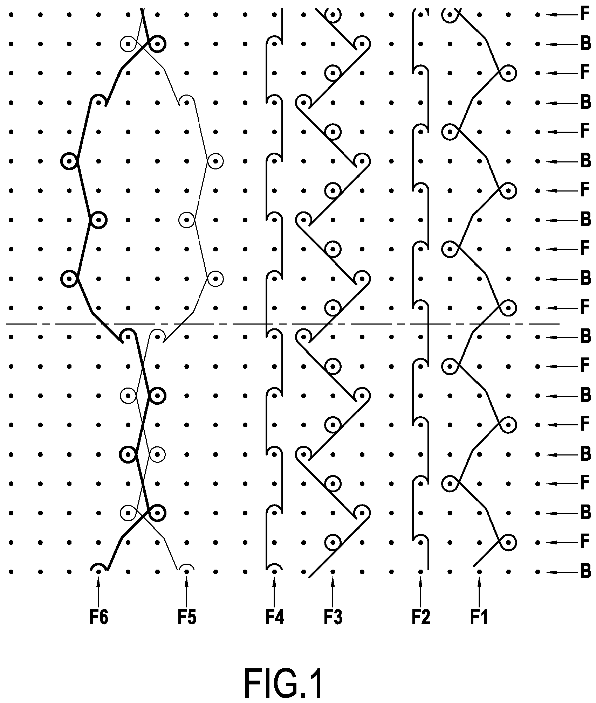

L'invention sera illustrée par les exemples et les tests comparatifs suivants, ainsi que par les figures 1, 2 et 3.

### Exemple de mise en œuvre de l'invention

On a fabriqué un tricot d'environ 10 cm de largeur selon l'invention sur un métier Raschel maille jetée double fonture jauge 22.

Pour réaliser le tricot on a utilisé 6 barres selon le schéma de maille représenté sur la figure 1 avec les fils et les conditions suivantes :

### Nature des fils

- F1: fil de polyamide commercialisé par la société RADICI sous la référence 78/18/1 dtex S Beige
- F2: fil d élasthanne de 44 dtex commercialisé par la société ASAHI KASEI GROUP
- F3 : fil qui est un monofilament de polyester de 55 dtex commercialisé par la société FILVA
- F4: fil d'élasthanne de 44 dtex commercialisé par la société ASAHI KASEI GROUP
- F5: fil de polyamide 66 commercialisé par la société EMILE TARDY sous la référence PA 66 1/ 44/34/ FT BE MM
- F6: fil de polyamide 66 commercialisé par la société EMILE TARDY sous la référence PA 66 1/ 44/34/ FT BE MM

### Réglages du métier à tricoter

- F1: alimentation 2500 mm de fils consommés pour la réalisation de 480 mailles - enfilage plein
- F2: alimentation 1500mm de fils consommés pour la réalisation de 480 mailles - enfilage 1plein/1 vide
- F3: alimentation 3500 mm de fils consommés pour la réalisation de 480 mailles - enfilage plein
- F4: alimentation 1600mm de fils consommés pour la réalisation de 480 mailles - enfilage 1plein/1 vide
- F5: alimentation 2250 mm de fils consommés pour la réalisation de 480 mailles- enfilage 3pleins/1vide
- F6 : alimentation 2250 mm de fils consommés pour la réalisation de 480 mailes -enfilage 3pleins/1vide

### Schéma de maille

On représente sur la figure 1 le graphique d'un exemple de structure de maille pour la réalisation d'un tricot selon un mode de réalisation particulier de l'invention.

Sur cette figure, on représente la fronture avant par la référence F, et la fronture arrière par la référence B. Les schémas de maille des fils F1 à F6 sont ensuite illustrés.

On comprend bien que cet exemple ainsi que les exemples suivant sont purement illustratifs, et qu'ils ne doivent pas être interprétés de manière limitative quant à la portée de l'invention.

Le tricot ainsi réalisé subit une étape de traitement par la chaleur en ligne.

Durant cette étape le tricot passe en une seule fois entre 2 rouleaux formés de cylindres chauffants de manière à ce que chaque face subisse ce traitement à la vitesse de 5,5 mètres par minute. La température des cylindres avant le passage du tricot est réglée pour être de l'ordre de 190 °C.

Les techniques suivantes ont été utilisées pour évaluer les paramètres du tricot obtenu.

### Mesure du grammage

La mesure du grammage est réalisée selon la norme NF EN 12127. On pèse 5 éprouvettes d'une surface de 100 cm² (mesure = +/- 1 %) avec une balance dont la précision est au plus de 1 mg.

La pesée est réalisée à une température de 21 °C +/-2 °C et 60 % +/- 15 % HR.

La mesure finale est une moyenne des 5 éprouvettes.

### Mesure de l'épaisseur

La mesure de l'épaisseur s'effectue selon la norme NF EN ISO 9073-2. On utilise un micromètre laser KEYENCE, (muni d'une tête de capteur laser CCD LK-G87 et d'un capteur de déplacement laser CCD LK-G3001PV). La pression d'application est fixée à 0,5kPa et la surface du disque en acier est de 2500mm².

### Mesure de l'écart entre les faces

Cette mesure est réalisée de la façon suivant.

A l'aide d'un microscope numérique KEYENCE (optiques x 100 ou x 200) on détermine l'espace entre les deux plans des 2 surfaces textiles.

Le plan moyen des 2 surfaces est matérialisé par un trait horizontal estimé par l'opérateur et la distance entre les deux traits est déterminée de manière automatique par le logiciel .La mesure est reproduite plusieurs fois afin d'augmenter la précision et on réalise une moyenne des mesures obtenues.

### Mesure de la contrainte seuil de cisaillement

Les mesures sont réalisées à l'aide d'un rhéomètre DHR2 commercialisé par la société TA Instruments.

Elles sont effectuées à une température de 35°C (de manière à être proche de la température des bandes au contact de la peau) ladite température étant régulée par un plan Peltier qui équipe le rhéomètre. On découpe 2 disques de 25 mm de diamètre du tricot 3D analysé.

Ces 2 disques sont collés respectivement à l'aide d'un adhésif double face fin et rigide commercialisé par la société Plasto sous la référence P753 sur la face métallique du plateau mobile et du plateau du plan Peltier du rhéomètre. On met en contact les 2 disques de tricot 3D, face structure charmeuse (également appelée structure drap) sur face structure filet, en appliquant une pression de 5,3 kPa (soit l'équivalent de 40mm de mercure). Le programme de pilotage du rhéomètre génère une rampe de contrainte (couple de torsion) qui varie de 100 à 10 000 Pa en 600 secondes. L'appareil enregistre le premier micro-déplacement qu'il détecte qui correspond à la contrainte seuil de cisaillement exprimée en Pa.

On considère que l'incertitude instrumentale sur cette mesure est plus ou moins 6%.

Les paramètres du tricot obtenu sont les suivantes (Exemple 1) :
- Grammage : 232g/m²
- Epaisseur : 1,23 mm
- Contrainte seuil de cisaillement : 3080 Pa
- Ecart entre les faces : 0,64 mm
- Allongement longitudinal selon la norme EN 14704 - 1 : 56 %
- Allongement transversal selon la norme EN 14704 - 1 : 128 %

On a également réalisé plusieurs autres exemples de tricot, que l'on détaille ci-après.

Ces autres exemples sont réalisés au moyen d'un schéma de maille identique à celui détaillé pour l'Exemple 1 (sauf indication contraire). On détaille ci-après la nature des fils et le réglage du métier à tricoter, ainsi que les caractéristiques obtenues.

**Exemple 2** : correspondant par exemple à un produit ayant une face pleine au contact de la peau d'un sujet, et une face opposée qui est un filet :

### Nature des fils :

- F1: fil de polyamide commercialisé par la société RADICI sous la référence 78/24/1 dtex S Beige
- F2: fil d'élasthanne de 44 dtex commercialisé par la société ASAHI KASEI GROUP
- F3 : fil qui est un monofilament de polyester de 55 dtex commercialisé par la société FILVA
- F4: fil d'élasthanne de 44 dtex commercialisé par la société ASAHI KASEI GROUP
- F5: fil de polyamide 66 commercialisé par la société EMILE TARDY sous la référence PA 66 1/ 44/34/ FT BE MM
- F6: fil de polyamide 66 commercialisé par la société EMILE TARDY sous la référence PA 66 1/ 44/34/ FT BE MM

### Réglage du métier à tricoter :

- F1: alimentation 2000 mm de fils consommés pour la réalisation de 480 mailles - enfilage plein
- F2: alimentation 1500 mm de fils consommés pour la réalisation de 480 mailles - enfilage 1 plein / 1 vide
- F3: alimentation 3500 mm de fils consommés pour la réalisation de 480 mailles - enfilage plein
- F4: alimentation 1600mm de fils consommés pour la réalisation de 480 mailles - enfilage 1 plein / 1 vide
- F5: alimentation 2150 mm de fils consommés pour la réalisation de 480 mailles- enfilage 3 pleins / 1 vide
- F6 : alimentation 2250 mm de fils consommés pour la réalisation de 480 mailes -enfilage 3 pleins / 1 vide

Le tricot ainsi réalisé subit une étape de traitement par la chaleur en ligne.

Durant cette étape le tricot passe en une seule fois entre 2 rouleaux formés de cylindres chauffants de manière à ce que chaque face subisse ce traitement à la vitesse de 5,5 mètres par minute. La température des cylindres avant le passage du tricot est réglée pour être de l'ordre de 190 °C.

### Caractéristiques du produit obtenu :(Exemple 2)

- Grammage : 231g/m²
- Epaisseur : 1,22 mm
- Contrainte seuil de cisaillement : 3027 Pa
- Ecart entre les faces : 0,52 mm
- Allongement longitudinal selon la norme EN 14704 - 1 : 62 %
- Allongement transversal selon la norme EN 14704 - 1 : 173 %

**Exemple 3:** correspondant par exemple à un produit qui est un allongement long :

### Nature des fils :

- F1: fil de polyamide 66 commercialisé par la société EMILE TARDY sous la référence PA 66 1/ 44/34/ FT BE MM
- F2: fil d'élasthanne de 44 dtex commercialisé par la société ASAHI KASEI GROUP
- F3 : fil qui est un monofilament de polyester de 55 dtex commercialisé par la société FILVA
- F4: fil d'élasthanne de 44 dtex commercialisé par la société ASAHI KASEI GROUP
- F5: fil de polyamide 66 commercialisé par la société EMILE TARDY sous la référence PA 66 1/ 44/34/ FT BE MM
- F6: fil de polyamide 66 commercialisé par la société EMILE TARDY sous la référence PA 66 1/ 44/34/ FT BE MM

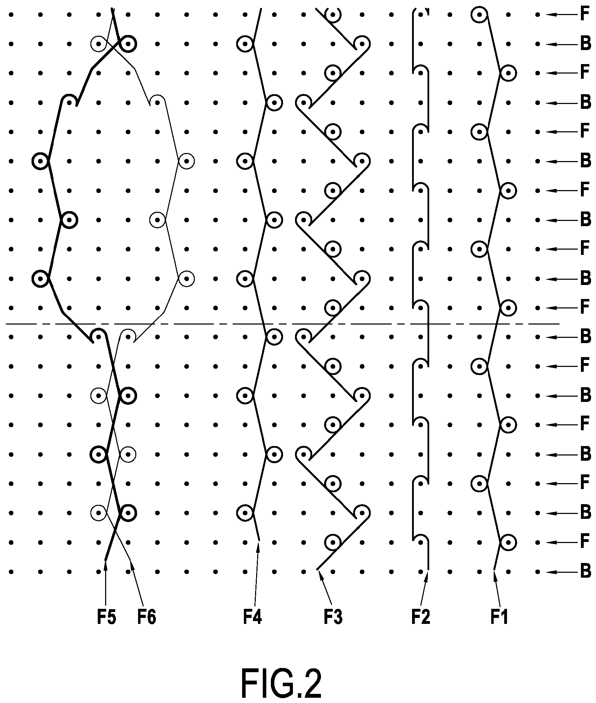

Le schéma de maille est ici distinct de celui des autres exemples, et est représenté sur la figure 2.

### Réglage du métier à tricoter :

- F1: alimentation 2100 mm de fils consommés pour la réalisation de 480 mailles - enfilage plein
- F2: alimentation 2050 mm de fils consommés pour la réalisation de 480 mailles - enfilage plein
- F3: alimentation 3700 mm de fils consommés pour la réalisation de 480 mailles - enfilage plein
- F4 : alimentation 2300 mm de fils consommés pour la réalisation de 480 mailles - enfilage plein
- F5: alimentation 2150 mm de fils consommés pour la réalisation de 480 mailles- enfilage 3 pleins / 1 vide
- F6 : alimentation 2150 mm de fils consommés pour la réalisation de 480 mailes -enfilage 3 pleins / 1 vide

Le tricot ainsi réalisé subit une étape de traitement par la chaleur en ligne.

Durant cette étape le tricot passe en une seule fois entre 2 rouleaux formés de cylindres chauffants de manière à ce que chaque face subisse ce traitement à la vitesse de 5,9 mètres par heure. La température des cylindres avant le passage du tricot est réglée pour être de l'ordre de 70 °C.

Une bande de produit ainsi obtenue a ensuite été soumise à 5 lavages successifs, sans séchage entre les lavages, dans une machine à laver à 40°C et 800 tours/minutes, avec une lessive commercialisée sous la marque commerciale « Le Chat machine ».

### Caractéristiques du produit obtenu :(Exemple 3)

- Grammage : 367/m²
- Epaisseur : 1,9 mm
- Contrainte seuil de cisaillement : 4077 Pa
- Ecart entre les faces : 1,08 mm
- Allongement longitudinal selon la norme EN 14704 - 1 : 111 %
- Allongement transversal selon la norme EN 14704 - 1 : 191 %

**Exemple 4:** correspondant par exemple à un produit ayant un fil d'entretoise de 22dtex :

### Nature des fils :

- F1: fil de polyamide commercialisé par la société RADICI sous la référence 78/18/1 dtex S Beige
- F2: fil d'élasthanne de 44 dtex commercialisé par la société ASAHI KASEI GROUP
- F3 : fil qui est un monofilament de polyester de 22 dtex commercialisé par la société FILVA
- F4: fil d'élasthanne de 44 dtex commercialisé par la société ASAHI KASEI GROUP
- F5: fil de polyamide 66 commercialisé par la société EMILE TARDY sous la référence PA 66 1/ 44/34/ FT BE MM
- F6: fil de polyamide 66 commercialisé par la société EMILE TARDY sous la référence PA 66 1/ 44/34/ FT BE MM

### Réglage du métier à tricoter :

- F1: alimentation 2400 mm de fils consommés pour la réalisation de 480 mailles - enfilage plein
- F2: alimentation 1600 mm de fils consommés pour la réalisation de 480 mailles - enfilage 1 plein / 1 vide
- F3: alimentation 3900 mm de fils consommés pour la réalisation de 480 mailles - enfilage plein
- F4 : alimentation 1600 mm de fils consommés pour la réalisation de 480 mailles - enfilage 1 plein / 1 vide
- F5: alimentation 2000 mm de fils consommés pour la réalisation de 480 mailles- enfilage 3 pleins / 1 vide
- F6 : alimentation 2000 mm de fils consommés pour la réalisation de 480 mailes -enfilage 3 pleins / 1 vide

Le tricot ainsi réalisé subit une étape de traitement par la chaleur en ligne.

Durant cette étape le tricot passe en une seule fois entre 2 rouleaux formés de cylindres chauffants de manière à ce que chaque face subisse ce traitement à la vitesse de 5,5 mètres par minute. La température des cylindres avant le passage du tricot est réglée pour être de l'ordre de 190 °C.

### Caractéristiques du produit obtenu :(Exemple 4)

- Grammage : 199/m²
- Epaisseur : 1,1 mm
- Contrainte seuil de cisaillement : 3007 Pa
- Ecart entre les faces :0,57 mm
- Allongement longitudinal selon la norme EN 14704 - 1 : 54 %
- Allongement transversal selon la norme EN 14704 - 1 : 192 %

**Exemple 5** : correspondant par exemple à un produit sans face ajourée c'est-à-dire avec 2 faces pleines :

### Nature des fils :

- F1: fil de polyamide commercialisé par la société RADICI sous la référence 78/18/1 dtex S Beige
- F2: fil d'élasthanne de 44 dtex commercialisé par la société ASAHI KASEI GROUP
- F3 : fil qui est un monofilament de polyester de 55 dtex commercialisé par la société FILVA
- F4: fil d'élasthanne de 44 dtex commercialisé par la société ASAHI KASEI GROUP
- F5: fil de polyamide 66 commercialisé par la société Defiber sous la référence PA 66 1/ 44/34/ dTex
- F6 : fil de polyamide 66 commercialisé par la société Defiber sous la référence PA 66 1/ 44/34/ dTex

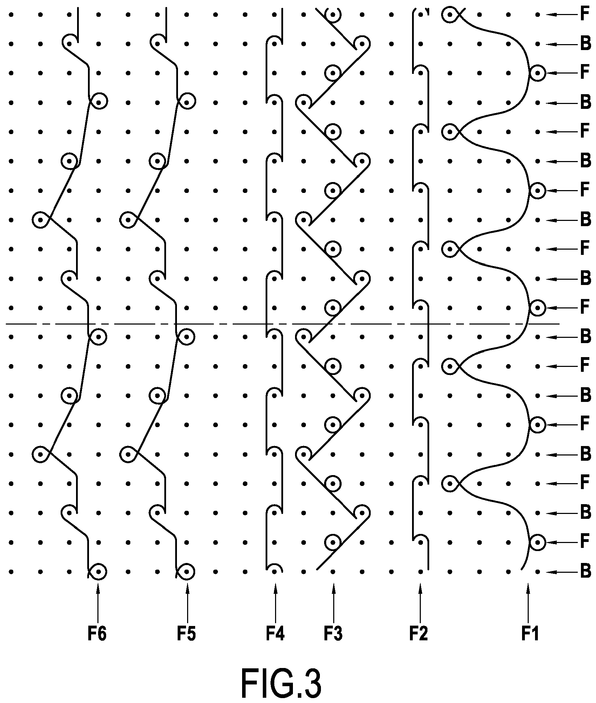

Le schéma de maille est ici distinct de celui des autres exemples, et est représenté sur la figure 3.

### Réglage du métier à tricoter :

- F1: alimentation 3200 mm de fils consommés pour la réalisation de 480 mailles - enfilage plein
- F2: alimentation 1600mm de fils consommés pour la réalisation de 480 mailles - enfilage 1plein/1 vide
- F3: alimentation 3500 mm de fils consommés pour la réalisation de 480 mailles - enfilage plein
- F4: alimentation 1700mm de fils consommés pour la réalisation de 480 mailles - enfilage 1plein/1 vide
- F5: alimentation 1900 mm de fils consommés pour la réalisation de 480 mailles- enfilage 3pleins/1vide
- F6 : alimentation 1900 mm de fils consommés pour la réalisation de 480 mailes -enfilage 3pleins/1vide

Le tricot ainsi réalisé subit ensuite une étape de traitement par la chaleur en ligne.

Durant cette étape le tricot passe en une seule fois entre 2 rouleaux formés de cylindres chauffants de manière à ce que chaque face subisse ce traitement à la vitesse de 5 mètres par minute. La température des cylindres avant le passage du tricot est réglée pour être de l'ordre de 165 °C.

### Caractéristiques du produit obtenu :(Exemple 5)

- Grammage : 262/m²
- Epaisseur : 1,2 mm
- Contrainte seuil de cisaillement : 3027 Pa
- Ecart entre les faces : 0,76 mm
- Allongement longitudinal selon la norme EN 14704 - 1 : 43 %
- Allongement transversal selon la norme EN 14704 - 1 : 127 %

On a ensuite comparé les performances de pression in vitro, selon le test décrit ci-après, entre les exemples selon l'invention et le système de contention bicouche commercialisés sous la dénomination K2 par la société Laboratoires URGO.

### Test in vitro

Les performances du tricot 3D des exemples 1 à 5 et du système de contention bicouche commercialisés sous la dénomination K2 par la société Laboratoires URGO ont été évaluées en termes de pressions de travail et de repos et de différentiel de pression, au cours du temps.

On a utilisé la méthode et l'appareil de test in vitro décrit dans la demande de brevet WO 2007/113430 page 17 ligne 26 à page 19 ligne 18. Selon cette méthode la bande est posée autour d'un cylindre avec un recouvrement total de 100%, puis on fait varier la circonférence du cylindre à une vitesse imposée de façon continue entre une position dite de repos (diamètre le plus faible) et une position dite de travail (diamètre le plus grand) pour mimer la contraction musculaire. Des capteurs de pression mesurent au cours du temps les valeurs des pressions de repos et des pressions de travail.

L'écart de temps entre les mesures de pression de travail et de pression de repos est de 5 secondes et la fréquence des mesures de ces deux paramètres successifs est de 0,2Hz.

Pour tester les bandes de contention selon l'invention on a déterminé l'allongement à la pose de la bande en fonction de la pression de travail recherchée, par exemple à l'aide de la courbe traction rupture telle que définie dans la norme EN ISO 13934-1. D'après la loi de Laplace, l'allongement à effectuer correspond à la pression recherchée.

On découpe une bande rectangulaire de largeur suffisante en l'effilochant si besoin pour obtenir un échantillon de largeur finale de 50mm. On place cet échantillon dans les mors d'un dynamomètre distants de 200mm. On procède à l'essai de traction jusqu'à la rupture de l'échantillon à la vitesse de 100mm/mn. On répète ainsi le test pour 5 échantillons. Les conditions de conditionnement, d'hygrométrie et de température sont définies dans la norme EN ISO 13934 - 1.

On a ainsi déterminé un allongement à la pose de 40% pour la bande selon les exemples 1 à 3, de 45% pour l'exemple 4 et de 30% pour l'exemple 5 de l'invention de manière à appliquer à la pose une pression maximale de l'ordre de 50 à 70 mm de mercure.

Pour poser la bande de façon appropriée, on a étalonné les bandes à l'aide d'un pochoir comme décrit dans la demande de brevet WO 2007/113340 page 13, ligne 18 à page 14, ligne 6.

Les résultats obtenus pour la bande obtenue selon les exemples de l'invention et le système de contention bicouches commercialisé par la société Laboratoires URGO sous la dénomination K2 taille 18-25 cm sont rassemblés dans les tableaux 1 et 2 ci-après.

La valeur «Pression Max à T0» correspond à la première pression de travail enregistrée immédiatement après la pose, et «Delta à T0»correspond au différentiel de pression entre la première pression de travail et la première pression de repos enregistrées immédiatement après la pose. Les valeurs «Pression Max à T24» et «Delta à T24» correspondent aux mesures enregistrées 24 heures après la pose, mesurées en mm de mercure. Puis on a calculé la différence entre T0 et T24 heures «Delta (T0 - T24h)».

**Tableau 1**

| Exemple 1 | Exemple 2 | K2 (Laboratoires URGO) | Mesure effectuée |
|---|---|---|---|
| 40% | 40% | 55% + 50% | Allongement à la pose |
| 69 | 63 | 44 | Pression max à T0 |
| 28 | 25 | 19 | Delta à T0 |
| 51 | 45 | 35 | Pression max à T24 |
| 25 | 21 | 17 | Delta à T24 |
| +3 | +4 | +2 | Delta (T0-T24) |

**Tableau 2**

| Exemple 3 | Exemple 4 | Exemple 5 | Mesure effectuée |
|---|---|---|---|
| 40% | 45% | 30% | Allongement à la pose |
| 52 | 71 | 50 | Pression max à T0 |
| 15 | 31 | 26 | Delta à T0 |
| 44 | 52 | 34 | Pression max à T24 |
| 21 | 26 | 20 | Delta à T24 |
| -6 | +5 | +6 | Delta (T0-T24) |

Ces tableaux montrent que l'on obtient des résultats en terme de pressions appliquées à 24 heures et de différentiel de pression à 24 heures à la fois pour le système bi- couches K2 et les mono bandes selon l'invention qui sont compris dans les fourchettes visées à savoir un pression maximale à 24 heures comprises entre 34 et 50 mm de mercure et un différentiel de pression à 24 heures entre 15 et 25 mm de mercure.

Les valeurs de différentiels de pression à 24 heures qui sont importantes pour l'efficacité du traitement sont mêmes supérieures pour les mono bandes selon l'invention, à savoir de 20 à 26 mm de mercure contre 17 mm de mercure pour le système bicouches K2. On constate aussi que pour tous les produits des exemples 1, 2, 4 et 5 et le produit K2, qui sont tous des allongements courts ce différentiel de pression varie peu au cours du temps puisque la variation est entre +3 et +6 pour les tricots selon l'invention et +/ 2 pour le système bicouches K2.

De façon surprenante l'exemple 3 qui est un allongement long, donc censé être moins efficace en termes de différentiel de pression, présente lui aussi un excellent différentiel de pression de 21mm de mercure. De plus ce différentiel de pression s'améliore au cours du temps et il est nettement meilleur à 24 heures qu'à T=0 ; 21 contre 15 mm de mercure.

En conclusion les bandes selon l'invention permettent d'obtenir des propriétés thérapeutiques équivalentes à celles du produit K2 voire même supérieures, et de conserver ces dernières au cours du temps et ceci avec une seule bande et sans ajout de latex ou d'adhésif.

De la même façon on a comparé sur le test in vivo décrit ci-après l'exemple 1, et le produit K2 pour évaluer le relâchement intrinsèque des bandes au cours du temps.

Le mode opératoire de ce test in vivo est le suivant.

On enroule les bandes autour de la jambe selon les préconisations décrites dans la notice du système bicouches K2.

Pour rappel, cette notice préconise la méthode d'application suivante :
1) Tenir le pied à 90°, orteils vers le haut. Appliquer KTECH à la base des orteils en faisant deux tous d'ancrage, en s'assurant que la face ouatée soit en contact direct avec la peau et que l'indicateur de pression soit situé sur le côté supérieur de la bande. Continuer en faisant une figure en « 8 » autour de la cheville, sans appliquer une tension excessive sur le pied et en recouvrant bien le talon.
2) Remonter jusqu'au genou en effectuant des spirales et en étirant la bande de manière appropriée : l'indicateur de pression imprimé sur les bandes doit former un cercle. Pour obtenir un recouvrement correct, l'indicateur de pression doit être tout juste recouvert (recouvrement de 50%). Finir 2cm sous le genou et couper l'excédent de bande. Fixer à l'aide d'un sparadrap.
3) Appliquer KPRESS sur KTECH selon la même technique en commençant un doigt au-dessus de KTECH et en finissant un doigt sous KTECH afin que seule KTECH soit en contact direct avec la peau. Une fois appliquée, appuyer doucement sur le bandage avec les mains pour assurer une bonne tenue du système.

On comprend bien que cette dernière étape 3) n'est pas nécessaire pour une bande de contention selon l'invention.

Pour l'exemple selon l'invention on utilise un allongement à la pose comme précédemment pour le test in vitro de 40 % et on étalonne de la même façon le tricot. On enroule les bandes autour du pied, du talon et le long de la jambe jusqu'au genou avec un recouvrement d'une couche sur l'autre de 50%. La dernière spire est fixée sur elle-même à l'aide d'une attache métallique ou d'un sparadrap. Si on souhaite vérifier la pression appliquée par la bande on peut disposer en un point B1, correspondant à la zone où le tendon d'Achille se transforme en muscle du mollet, soit de manière générale 10 à 15 cm environ au-dessus de la malléole, un capteur de mesure de pression d'interface comme par exemple le capteur référencée KKH-01 de la société KIKUHIME .On trace à l'aide d'un feutre fin et non effaçable un trait vertical sur au moins 3 spires, sur l'axe de la crête tibiale, à partir de la dernière spire enroulée. Cette marque sert de référence pour évaluer à l'aide d'un réglet gradué au mm, le décalage horizontal du trait au terme de la durée du test. Lors des mouvements, ce trait perd de son caractère rectiligne et se présente en échelons d'autant plus décalés que les glissements spires sur spires sont importants. Si le glissement spire sur spire est très faible ou inexistant, le trait vertical reste intègre ou varie très peu principalement sur la première spire qui se situe sous la dernière spire enroulée.

Ce décalage du trait vertical est représentatif du relâchement de la bande et illustre son glissement potentiel au cours du temps.

Ce test a été réalisé durant 6 heures sur 5 personnes. Chaque personne porte sur une jambe une bande selon l'exemple 1 de 10 cm de largeur et 2,6 m de longueur, face filet au contact de la peau, étalonnée à 40% à la pose et sur l'autre jambe le système bicouches K2.

On mesure au bout des 6 heures le décalage du trait vertical sur les 3 premières spires.

Les résultats sont les suivants :
Système bicouches K2 : aucun décalage du trait sur aucune spire.

Un tel résultat est cohérent, du fait de la « cohésivation » de la bande qui bloque le glissement des spires les unes sur les autres.

Bande de contention selon l'exemple 1: aucun décalage du trait sur les spires 2 et 3 et un léger décalage moyen de 4 mm sur la première spire qui se situe sous la dernière spire enroulée.

Cette valeur moyenne est négligeable et représente les aléas de mesures liés à la variation des mollets des testeurs, la reproductibilité de la pose et les variabilités de fabrication des bandes.

On peut donc considérer que la bande selon l'invention présente une résistance au glissement spire sur spire équivalente à celle du système cohésivé.

Ce test montre qu'en termes de tenue les 2 produits sont équivalents.

Selon le même protocole on a testé sur une seule personne la bande de l'exemple 5 étalonnée à 30% à la pause comme précédemment pour le test in vitro. La seule différence est que la dernière spire est fixée sur elle-même à l'aide de 2 bandes velcro à la place d'une attache métallique.

On constate à nouveau après une durée de 6 heures, un léger décalage du trait de 3 mm sur la première spire qui se situe sous la dernière spire enroulée. Un décalage de 1 mm est relevé sur la spire 2 (ce qui représente l'incertitude sur l'évaluation de la mesure de l'épaisseur du trait, et est donc négligeable), et on ne relève aucun décalage sur la spire 3.

On peut donc considérer que la bande de l'exemple 5 présente une résistance au glissement spire sur spire équivalente à celle de l'exemple 1.

L'ensemble de ces tests démontrent que l'on a bien obtenu avec une seule monobande un dispositif de contention qui présente les bonnes propriétés thérapeutiques et qui ne glisse pas au cours du temps sans l'ajout de substances supplémentaires sur cette dernière.

## Revendications

1. Bande de contention qui se présente sous la forme d'un tricot obtenu par la technologie maille jetée à base de fils synthétiques qui est constitué de 2 surfaces textiles dont la structure textile est identique ou différente, reliées entre elles par des fils d'entretoises (F3), chaque surface comportant des fils élastiques (F2, F4), edit tricot présentant
- un allongement longitudinal mesuré selon la norme EN 14704 -1 compris entre 30 et 160%,
**caractérisé en ce qu'**il présente une contrainte de seuil de cisaillement supérieure ou égale à 2800 Pa.

2. Bande de contention selon la revendication 1, dans lequelle le fil d'entretoise (F3) est un monofilament présentant un titre compris entre 20 et 80 dtex.

3. Bande de contention selon la revendication 2, dans lequel le fil d'entretoise (F3) présentant un titre compris entre 40 et 70 dtex.

4. Bande de contention selon l'une des revendications précédentes, dans laquelle ledit tricot présente une face qui a une structure textile parmi la liste ci-après,
- charmeuse ;
- demi-simple à mailles ouvertes ou fermées ;
- atlas sous un ou plusieurs rangs ;
- chainette à mailles ouvertes, fermées, ou à alternance de mailles fermées et ouvertes ;
ladite face étant opposée à la face adaptée pour être mise en contact avec la peau qui a une structure textile qui est un filet, présentant une structure textile ajourée.

5. Bande de contention selon l'une des revendications précédentes dans laquelle le tricot présente une épaisseur comprise entre 1 et 2 mm.

6. Bande de contention selon la revendication 5, dans laquelle le tricot présente une épaisseur comprise entre 1 et 1,5 mm.

7. Bande de contention selon l'une des revendications précédentes, dans laquelle le tricot présente un écart entre les faces compris entre 0,4 et 1,5mm.

8. Bande de contention selon l'une des revendications précédentes dans laquelle le tricot présente un grammage compris entre 160 et 370 g/m².

9. Bande de contention selon la revendication 8, dans laquelle le tricot présente un grammage compris entre 160 et 300 g/m².

10. Bande de contention selon l'une des revendications précédentes, dans laquelle le tricot présente un allongement longitudinal tel que défini dans la norme EN 14704-1 compris entre 50 et 120%.

11. Bande de contention selon l'une des revendications précédentes dans laquelle le tricot est fabriqué en utilisant une seule barre pour le fil d'entretoise (F3) qui relie les 2 faces textiles.

12. Bande de contention selon l'une des revendications précédentes dans laquelle le fil élastique (F2, F4) présente un titre compris entre 40 et 80 dtex.

13. Bande de contention selon l'une des revendications précédentes dans laquelle les surfaces textiles comprennent des fils thermoplastiques (F1, F5, F6) présentant des titres de 40 à 90 dtex.

14. Kit qui comprend une ou plusieurs bandes de contention selon l'une des revendications précédentes et un ou plusieurs pansements adaptés pour être disposés sur une plaie préalablement à la bande de contention.

## Patentansprüche

1. Kompressionsbandage, die die Form einer Maschenware aufweist, die durch die Maschenlegetechnologie auf Basis von synthetischen Fäden erhalten ist, die aus zwei Textiloberflächen gebildet ist, deren Textilstruktur identisch oder unterschiedlich ist, die durch Querfäden (F3) miteinander verbunden sind, wobei jede Oberfläche elastische Fäden (F2, F4) aufweist, wobei die Maschenware
- eine Längsdehnung, die gemäß der Norm EN 14704-1 gemessen ist, von zwischen 30 und 160 % aufweist,
**dadurch gekennzeichnet, dass** sie
- eine Grenzscherspannung von größer oder gleich 2.800 Pa aufweist.

2. Kompressionsbandage nach Anspruch 1, wobei der Querfaden (F3) ein Monofilament ist, das einen Titer von zwischen 20 und 80 dtex aufweist.

3. Kompressionsbandage nach Anspruch 2, wobei der Querfaden (F3) einen Titer von zwischen 40 und 70 dtex aufweist.

4. Kompressionsbandage nach einem der vorhergehenden Ansprüche, wobei die Maschenware eine Seite aufweist, die eine Textilstruktur von der nachfolgenden Liste aufweist:
- Charmeuse,
- offene oder geschlossene Halbmasche,
- Atlas unter einer oder mehreren Reihen,
- Franse mit offener Legung, geschlossener Legung oder mit Wechsel von geschlossener und offener Legung,
wobei die Seite der Seite entgegengesetzt ist, die dazu ausgelegt ist, mit der Haut in Kontakt gebracht zu werden und die eine Textilstruktur aufweist, die ein Netz ist, das eine durchbrochene Textilstruktur aufweist.

5. Kompressionsbandage nach einem der vorhergehenden Ansprüche, wobei die Maschenware eine Dicke zwischen 1 und 2 mm aufweist.

6. Kompressionsbandage nach Anspruch 5, wobei die Maschenware eine Dicke zwischen 1 und 1,5 mm aufweist.

7. Kompressionsbandage nach einem der vorhergehenden Ansprüche, wobei die Maschenware einen Abstand zwischen 0,4 und 1,5 mm zwischen den Seiten aufweist.

8. Kompressionsbandage nach einem der vorhergehenden Ansprüche, wobei die Maschenware ein Flächengewicht zwischen 160 und 370 g/m² aufweist.

9. Kompressionsbandage nach Anspruch 8, wobei die Maschenware ein Flächengewicht zwischen 160 und 300 g/m² aufweist.

10. Kompressionsbandage nach einem der vorhergehenden Ansprüche, wobei die Maschenware eine gemäß der Norm EN 14704-1 definierte Längsdehnung zwischen 50 und 120 % aufweist.

11. Kompressionsbandage nach einem der vorhergehenden Ansprüche, wobei die Maschenware unter Verwendung einer einzigen Barre für den Querfaden (F3) hergestellt ist, der die beiden Textilseiten verbindet.

12. Kompressionsbandage nach einem der vorhergehenden Ansprüche, wobei der elastische Faden (F2, F4) einen Titer von zwischen 40 und 80 dtex aufweist.

13. Kompressionsbandage nach einem der vorhergehenden Ansprüche, wobei die Textiloberflächen thermoplastische Fäden (F1, F5, F6) umfassen, die Titer von 40 bis 90 dtex aufweisen.

14. Kit, das eine oder mehrere Kompressionsbandagen nach einem der vorhergehenden Ansprüche und einen oder mehrere Verbände umfasst, der/die geeignet ist/sind, vor der Kompressionsbandage auf einer Wunde angeordnet zu werden.

## Claims

1. A compression bandage in the form of a knit obtained by warp stitch technology on the basis of synthetic yarns and comprising two textile surfaces of respective textile structures that may be identical or different, the surfaces being interconnected by spacer yarns (F3), each surface including elastic yarns (F2, F4), said knit presenting:
- longitudinal stretch measured in compliance with the standard EN 14704-1 lying in the range 30% to 160%; **characterized in that** it presents:
- threshold shear stress greater than or equal to 2800 Pa.

2. A compression bandage according to claim 1, wherein the spacer yarn (F3) is a monofilament presenting weight lying in the range 20 dtex to 80 dtex.

3. A compression bandage according to claim 2, wherein the spacer yarn (F3) presents weight lying in the range 40 dtex to 70 dtex.

4. A compression bandage according to any preceding claim, wherein the knit presents a face that has a textile structure selected from the following list:
- charmeuse;
- open or closed loop single knit;
- atlas in one or more rows;
- closed or open loop or alternating closed loop and open loop pillar stitch;
said face being opposite to the face that is to be put into contact with the skin, which face has a textile structure that is a net, presenting an open-work textile structure.

5. A compression bandage according to any preceding claim, wherein the knit presents thickness lying in the range 1 mm to 2 mm.

6. A compression bandage according to claim 5, wherein the knit presents thickness lying in the range 1 mm to 1.5 mm.

7. A compression bandage according to any preceding claim, wherein the knit presents spacing between the faces lying in the range 0.4 mm to 1.5 mm.

8. A compression bandage according to any preceding claim, wherein the knit presents weight lying in the range 160 g/m² to 370 g/m².

9. A compression bandage according to claim 8, wherein the knit presents weight lying in the range 160 g/m² to 300 g/m².

10. A compression bandage according to any preceding claim, wherein the knit presents longitudinal stretch as defined in the standard EN 14704-1 lying in the range 50% to 120%.

11. A compression bandage according to any preceding claim, wherein the knit is fabricated using a single bar for the spacer yarn (F3) that connects together the two textile faces.

12. A compression bandage according to any preceding claim, wherein the elastic yarn (F2, F4) presents weight lying in the range 40 dtex to 80 dtex.

13. A compression bandage according to any preceding claim, wherein the textile surfaces include thermoplastic yarns (F1, F5, F6) presenting weights in the range 40 dtex to 90 dtex.

14. A kit comprising one or more compression bandages according to any preceding claim and one or more dressings adapted to be placed on a wound prior to putting the compression bandage into place.
